# FASCICULE DE BREVET EUROPEEN

(11) **EP 1 194 071 B1**
(45) Date de publication et mention de la délivrance du brevet: **02.11.2006**
(21) Numéro de dépôt: 00949664.7
(22) Date de dépôt: 06.07.2000
(51) Int. Cl.: A61B 8/04, A61B 5/022

(54) **PROCEDE ET DISPOSITIF POUR MESURER LA PRESSION SANGUINE**
VERFAHREN UND VORRICHTUNG ZUR BLUTDRUCKMESSUNG
METHOD AND DEVICE FOR MEASURING BLOOD PRESSURE

(30) Priorité: 08.07.1999 FR 9909131
(43) Date de publication de la demande: 10.04.2002
(73) Titulaire: BV Sport, 38600 Fontaine (FR)
(72) Inventeur: COUZAN, Serge, F-42000 Saint-Etienne (FR); PRUFER, Michael, F-06240 Beausoleil (FR)
(74) Mandataire: Maureau, Philippe
(86) Numéro de dépôt international: PCT/FR2000/001956
(87) Numéro de publication internationale: WO 2001/003584

(56) Documents cités:
- EP-A- 0 299 827
- EP-A- 0 420 085
- WO-A-98/55072
- FR-A- 2 481 918

## Description

Procédé et dispositif pour mesurer la pression sanguine.

L'invention est relative à un procédé et à un dispositif pour mesurer la pression sanguine, c'est-à-dire la pression veineuse et la pression artérielle.

Actuellement, la mesure de la seule pression artérielle par méthode indirecte est assurée par un sphygmomanomètre constitué par un brassard pneumatique apte à entourer le membre dont le circuit sanguin est soumis à la mesure. Ce brassard est équipé d'une poche en caoutchouc, élastiquement déformable, qui entoure complètement le segment de membre et est protégé de l'éclatement grâce à une sangle inextensible. Le brassard est relié à un moyen de gonflage, sous une pression plus ou moins grande, équipé d'un système de robinet avec possibilité de fermeture et d'ouverture mettant ainsi l'enveloppe à l'échappement dans l'atmosphère et permettant le gonflage et le dégonflage. L'ensemble est relié à un manomètre permettant de connaître la valeur des pressions exercées sur le membre.

Dans une première phase de la mesure de la pression artérielle, l'enveloppe est gonflée jusqu'à écraser complètement l'artère et arrêter le flux sanguin.

Dans une deuxième phase, l'opérateur procède à une décompression lente en dégonflant l'enveloppe, ce qui diminue la pression exercée par le brassard sur le membre et donc sur les parois de l'artère.

A partir d'une certaine pression, le passage du flux sanguin est possible et peut être détecté. Le nombre lu sur le manomètre et mémorisé correspond à la pression artérielle systolique.

Actuellement, les différentes techniques de mesure sont palpatoires (mesure du pouls), auscultatoires (avec un stéthoscope) ou ultrasonographiques (avec une sonde ultrasonique). Avec ces techniques, la mesure s'effectue en aval de la zone comprimée. On connaît aussi des appareils oscillométriques, (par exemple électroniques), avec mesure de la pression au niveau de la zone comprimée.

Les valeurs des mesures sont variables en fonction de nombreux facteurs (rythme circadien, position, effort, stress, digestion, ...), en fonction du membre examiné, la pression des membres inférieurs étant supérieure à celle des membres supérieurs et différentes selon la technique de mesure utilisée.

II existe également une confusion entre les termes de tension et de pression artérielle.

La pression artérielle est la pression qui, exercée par le sang sur les parois périphériques des artères, oscille au cours de la révolution cardiaque entre une valeur maximale, correspondant à l'arrivée de l'ondée systolique, et une valeur minimale, correspondant à la diastole. Cette pression artérielle est un concept physique.

La tension artérielle est plus un concept biologique. Elle représente la force élastique exercée par les parois artérielles sur le contenu sanguin pour contrebalancer la pression intramurale.

La définition de la pression artérielle normale est difficile car elle repose sur des données statistiques ou anatomocliniques. De plus, les valeurs sont plus ou moins précises selon la technique de mesure utilisée et ce mode de mesure indirect ne permet une analyse des pressions que sur certaines parties du membre (pli du coude, poignet, cheville).

Un autre inconvénient de ces techniques de mesure est qu'elles ne permettent pas de mesurer la pression sanguine d'une veine car elles ne peuvent détecter, de manière suffisamment précise, les modifications du flux veineux.

On connaît par ailleurs des échographes mettant en oeuvre une sonde émettrice réceptrice déplaçable manuellement directement sur la partie du corps humain devant être exploré, comme décrit dans EP-A-299 827, ou sur la fenêtre optique d'un brassard gonflable, comme dans WO98/55072. La sonde émet un flux ultrasonore et reçoit un spectre qui est traité par des moyens d'analyse et de calcul le transformant en signaux électriques. Sur un écran de lecture, ces signaux forment une cartographie en deux dimensions représentant le vaisseau analysé. Si certains échographes, tels celui décrit dans le document WO98/55072, peuvent aussi être utilisés pour aider à la réalisation de certains traitements, ils ne permettent pas de mesurer la valeur d'une pression sanguine, artérielle ou veineuse.

Le document EP-A-847 724 décrit un appareil de mesure des valeurs, respectivement maximale et minimale de la pression sanguine dans une artère et comprenant un brassard avec poche gonflable, placé exclusivement sur la partie supérieure du bras et non sur le membre inférieur, équipé de capteurs optiques, des moyens de traitement des signaux provenant des capteurs et générant sur un écran de visualisation un graphe représentant la variation de pression sanguine, un analyseur d'écran détectant les valeurs maximale et minimale de la pression sanguine et réagissant sur les moyens de gonflage de la poche du brassard. Cet appareil permet de mesurer, de manière indirecte à partir de l'examen d'une onde de pression, que les valeurs extrêmes de la pression dans une artère et ne peut pas mesurer une pression intermédiaire ou partielle dans cette artère et, encore moins dans une veine, puisque dans celle ci il n'y a pas de pulsation sanguine.

II s'avère de plus en plus que la connaissance de l'une à l'autre des pressions artérielles ou veineuses, en diverses zones précises d'un vaisseau sanguin, serait intéressante à des fins d'analyse, scientifique ou clinique, ou pour obtenir des résultats intermédiaires permettant indirectement de prendre une décision au sujet d'un traitement médical ou chirurgical, ou plus simplement de prendre connaissance de l'état de santé de la personne examinée.

La présente invention a pour objet de fournir un procédé permettant de mesurer la pression sanguine d'un vaisseau et comprenant, de façon connue, une phase de mise en compression du vaisseau sanguin par un moyen fluidique associé à un moyen de mesure de la pression du fluide, une phase de décompression lente, et une phase de mesure.

Selon l'invention, le procédé de mesure comprend également :
- avant la phase de mise en compression du vaisseau sanguin, une phase de repérage d'une image en au moins deux dimensions du vaisseau, l'image étant générée sur l'écran de lecture d'un appareil d'observation du corps humain par ondes réfléchies ou rayonnements traversants,
- et, pendant l'une des phases, respectivement de compression ou de décompression de ce vaisseau, une phase de repérage à l'écran d'une image en au moins deux dimensions d'un état particulier du vaisseau, cette phase déclenchant, dès que le vaisseau sanguin est dans cet état particulier, la phase de mesure de la pression des moyens fluidiques, opposée à la pression sanguine dans le vaisseau.

Ce procédé permet donc de mesurer, ponctuellement, dans une zone précise d'un vaisseau sanguin, la valeur d'une pression sanguine correspondant à un état du vaisseau et de répéter la mesure en plusieurs points d'un même vaisseau sanguin par simple déplacement des moyens de compression.

Une telle mesure peut être réalisée sur les artères et sur les veines ou toute partie du corps pouvant être comprimée localement par un moyen fluidique, tel qu'un brassard.

Selon les formes de mise en oeuvre, la pression mesurée est celle correspondant à l'un des états de référence suivants du vaisseau :
- vaisseau en début d'occlusion par compression par la poche gonflable du brassard,
- vaisseau comprimé à la reprise de la pulsabilité précédant son ouverture,
- vaisseau comprimé en début d'ouverture.

La phase de mesure de cette pression est réalisée après repérage à l'écran de lecture de l'état de référence correspondant, ce repérage déclenchant soit une intervention humaine de lecture de la pression sur le manomètre du brassard, soit un processus de mesure automatique de la pression dans la poche gonflable du brassard, donc de la pression sanguine équilibrée par la pression de la poche à l'instant de la mesure.

Dans une forme de mise en oeuvre particulière, le procédé comprend, après une mesure de la pression d'occlusion du vaisseau sanguin et à la fin d'une phase de décompression lente, une phase de repérage à l'écran d'une image en au moins deux dimensions du vaisseau observé, une phase de compression du vaisseau jusqu'à réduction de son calibre d'une valeur définie, et une phase de mesure de la pression instantanée des moyens de compression fluidique, déclenchée quand le vaisseau atteint le calibre attendu.

Cette application particulière permet, pour une réduction de valeur définie, en général d'un tiers, du calibre du vaisseau, c'est-à-dire de sa section transversale, de connaître l'accroissement de la valeur de pression sanguine par rapport à celle d'un vaisseau au calibre normal, mais aussi, par le calcul ou par une mesure complémentaire, de connaître la vitesse de circulation du sang, donc d'obtenir des données facilitant un diagnostic, l'adaptation d'un traitement, de même que la réalisation de moyens pour ce traitement, tel que manchon de contention.

Les phases de mesure d'un vaisseau sanguin dans divers états de référence peuvent être réalisées unitairement ou successivement sur une même zone du corps humain et, en particulier d'un membre. ,

Dans une forme de mise en oeuvre, le procédé consiste à mémoriser la valeur de chaque mesure de pression, à recommencer l'opération de mesure en plusieurs zones successives du membre examiné, et en fin de mesure, à appeler à l'écran les différentes valeurs mémorisées pour dresser une cartographie du vaisseau dans le membre examiné, et à mémoriser cette cartographie.

Cette cartographie est particulièrement intéressante pour la détection et l'adaptation du traitement des maladies artérielles et veineuses.

Lorsque l'appareil d'observation du corps humain est un échographe DOPPLER avec écran de lecture en couleur, procurant une représentation en deux ou trois dimensions, et est équipé de puissants moyens de traitement des données, le flux du vaisseau apparaît en couleur avec possibilité de détecter la direction et la vitesse du flux sanguin et, dans certaines applications, son débit.

L'invention concerne également un dispositif de mesure de la pression des vaisseaux sanguins.

De façon connue, ce dispositif comprend un brassard apte à entourer le membre dont le circuit sanguin est soumis à la mesure, ce brassard étant muni, d'une part, d'une poche gonflable raccordée à une source de fluide sous pression par un circuit de fluide équipé d'un moyen de mise à l'échappement et d'un moyen de mesure de la pression du fluide, et d'autre part, d'une fenêtre acoustique qui, réalisée dans un matériau perméable aux ondes ou rayonnements d'un appareil d'observation du corps humain, est apte à être appliquée sur la zone du membre comportant le vaisseau sanguin dont il faut mesurer la pression, ledit appareil étant équipé de moyens de traitement des signaux réfléchis et d'un écran de visualisation.

Selon l'invention, les moyens de traitement de l'appareil d'observation comprennent des moyens générant sur l'écran une image en au moins deux dimensions de la zone du vaisseau sanguin soumis aux ondes ou rayonnement de l'appareil d'observation et un analyseur d'images apte a) à détecter sur l'écran une image proche d'une image mémorisée correspondant à un état du vaisseau sanguin, tel que début de l'occlusion par compression, compression avec début de pulsabilité, reprise du flux sanguin, mise au calibre du vaisseau, et b) à commander le fonctionnement d'un appareil de mesure de la pression du fluide dans la poche gonflable.

Dans ces conditions, la mesure de la pression à l'état de référence du vaisseau s'effectue automatiquement et peut bénéficier de la précision et des moyens de mémorisation équipant l'appareil d'observation.

Dans une autre forme d'exécution, ce dispositif comprend :
- un boîtier électronique de gestion et de commande réagissant à un organe de déclenchement d'un cycle de mesure, ce cycle comprenant une ou plusieurs mesures successives mais relatives chacune à un état différent du vaisseau,
- un distributeur de fluide piloté par le boîtier de commande et régissant l'alimentation en fluide de la poche gonflable en la mettant en relation soit avec une source de fluide sous pression, soit avec un circuit d'échappement,
- un analyseur d'images envoyant au boîtier de commande, et quand le vaisseau atteint l'état de référence, un signal déclenchant une phase de la mesure,
- un appareil de mesure de la pression, piloté par le boîtier de commande pour mesurer, dans chaque phase de mesure, la pression dans la poche gonflable,
- et des moyens mémorisant la valeur de la pression mesurée.

Avec ce dispositif, les différentes phases et séquences de mesure s'effectuent de manière entièrement automatique après que le brassard ait été mis en place, le vaisseau repéré sur l'écran et l'organe de déclenchement actionné.

L'invention sera mieux comprise à l'aide de la description qui suit en référence au dessin schématique annexé représentant à titre d'exemple une forme d'exécution du dispositif selon l'invention, lorsqu'il est mis en oeuvre avec un échographe DOPPLER.
Figure 1 est une vue en perspective d'une forme d'exécution du brassard à fenêtre acoustique.
Figure 2 est une vue schématique des moyens mis en oeuvre pour effectuer une mesure de pression sanguine au moyen du brassard et d'un échographe.
Figures 3 et 4 sont des vues similaires à la figure 2 mais montrant un dispositif, respectivement, de mesure semi automatique et de mesure automatique.

Dans ce dessin, la référence numérique 1 désigne de manière générale, un brassard composé, de façon connue, d'une nappe 2 munie à ses extrémités, des moyens d'accrochage, tels que des bandes 3 de ruban à crochets aptes à coopérer avec des bandes 4 de ruban à bouclettes. Ce brassard est solidaire d'une poche gonflable 5 disposée contre sa face devant venir en contact avec la peau. Cette poche est reliée, par un conduit 6, en caoutchouc ou équivalent, à une poire de gonflage 7 équipée d'un robinet 8 et d'un manomètre 9.

La nappe 2 et la poche gonflable 5 sont remplacées, dans leur partie centrale, par une fenêtre acoustique 10, par exemple de forme carrée ou rectangulaire, dont les bords sont liés à elles, par exemple par couture et/ou collage. Cette fenêtre est réalisée dans un matériau synthétique souple, perméable aux ultrasons et ne générant pas de parasite lors d'une mesure ultrasonore.

Ce brassard fait partie d'un dispositif de mesure de la pression d'un vaisseau sanguin comportant également un échographe 20 dont la sonde 21 est reliée à un générateur d'ultrasons 22, par un circuit 23, et est reliée, par un circuit 24, à un récepteur 25 du spectre réfléchi par cette sonde. L'émetteur 22 et le récepteur 25 sont reliés à une unité de traitement 26, qui compare les signaux émis avec les signaux reçus et délivre, à un écran de lecture 27, une image 28 du vaisseau observé.

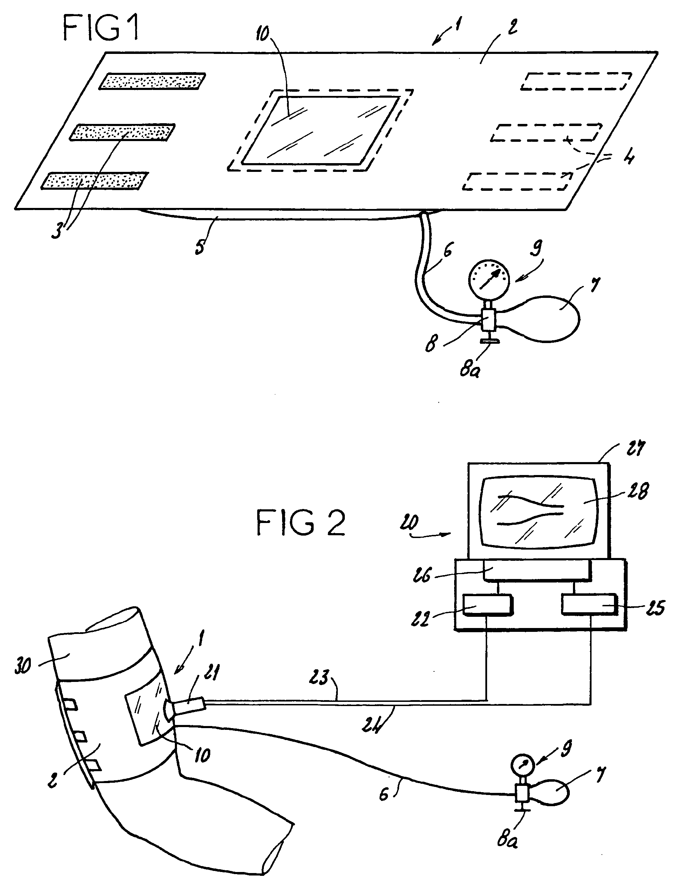

Pour effectuer une mesure de la tension sanguine d'un vaisseau, il faut d'abord mettre en place le brassard 1 autour du membre et par exemple autour du bras 30, en prenant soin de disposer la fenêtre acoustique 10 au-dessus de la zone contenant le vaisseau devant être analysé. De façon connue, la face de la peau et le dessus de la fenêtre acoustique sont recouverts d'un gel d'interface ou tout autre moyen permettant la bonne pénétration des ultrasons. La sonde 21 est alors appliquée sur la fenêtre 10 et déplacée jusqu'à ce qu'apparaisse à l'écran 27 l'image 28 du vaisseau soumis à la mesure. A ce stade, la poire 7 est actionnée pour gonfler la poche élastique 5. Ce gonflage s'effectue en surveillant visuellement l'image 28 jusqu'à ce que celle-ci fasse apparaître l'arrêt de circulation sanguine dans le vaisseau comme schématisé à la figure 2.

Dans la phase suivante de décompression lente, l'opérateur actionne la vis 8a de mise à l'échappement du robinet 8, de façon à procéder au dégonflage de l'enveloppe 5 et réduire la pression sur le membre 30. Dès que l'image 28 fait apparaître la pulsation sanguine précédant l'ouverture du vaisseau, la pression du manomètre 9 est lue et mémorisée.

En observant l'image 28, l'opérateur peut également procéder, d'une part, pendant la phase de mise en compression, à une mesure préalable de la pression lors de l'occlusion par compression du vaisseau, et d'autre part, pendant la phase de décompression lente, à des mesures complémentaires de pression sanguine pour divers états de référence, tels que vaisseau comprimé à la reprise de la pulsabilité, vaisseau en début d'ouverture, mais aussi, lors d'une nouvelle phase de compression à une mesure de pression quand le vaisseau atteint un calibre donné. On peut ainsi définir des pressions sanguines pour une réduction de calibre d'un quart, d'un tiers, d'un demi ou plus, qui, soit sont comparées à une pression sanguine de référence, par exemple celle du vaisseau sans compression, soit sont analysées par référence au débit sanguin mesuré par les moyens de traitement de l'appareil pour obtenir la vitesse de circulation sanguine, soit, enfin, sont associées à une vitesse de circulation sanguine mesurée par l'appareil, pour orienter le diagnostic et le traitement.

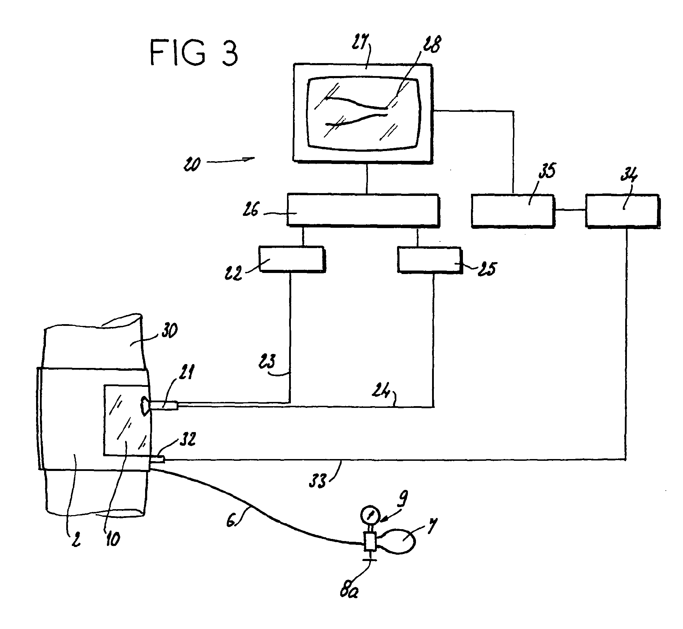

La forme d'exécution du dispositif représenté à la figure 3 est destinée à assurer une mesure semi automatique sans aucune intervention humaine autre que celle consistant à gonfler et dégonfler la poche 5 au moyen de la poire 7. A cet effet, l'enveloppe 5 est muni d'un capteur de pression 32 qui est relié par un circuit électrique 33 à un appareil de mesure 34 dont le fonctionnement est sous le contrôle d'un analyseur d'image 35.

Ainsi dans une phase de mesure de la pression pour un état de référence du vaisseau, par exemple de mesure de la pression sanguine à la reprise de la pulsabilité, dès que l'image 28 fait apparaître les pulsations préparatoires à l'ouverture du vaisseau, l'analyseur d'image 35 qui compare l'image à l'écran avec une image mémorisée, réagit sur l'appareil de mesure 34, qui mesure la pression fournie par le capteur 32 et mémorise cette valeur.

L'analyseur peut ensuite déclencher une nouvelle phase de mesure, par exemple celle de la pression sanguine au retour du flux sanguin.

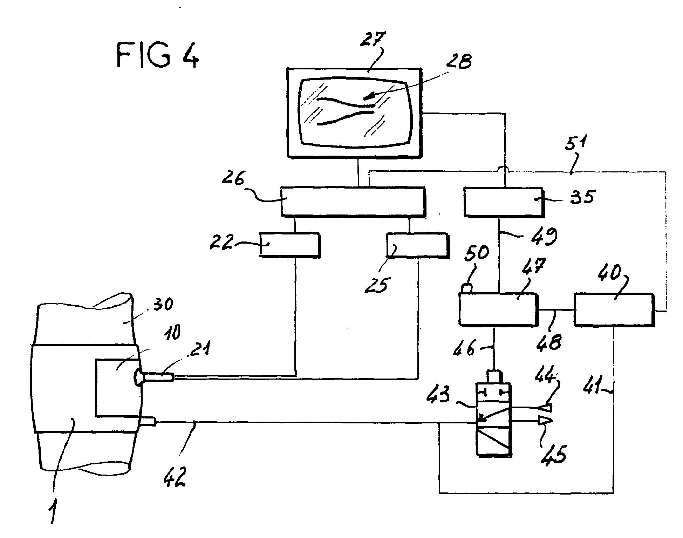

La forme d'exécution représentée à la figure 4 concerne un dispositif de mesure automatique. Il se différencie du précédent par le fait que l'appareil de mesure de la pression 40 est relié par un circuit pour fluide 41 à un circuit pour fluide 42 allant à la poche gonflable 5. Le circuit 42 est relié par un distributeur 43, soit à une source 44 de fluide sous pression et par exemple d'air ou d'un liquide, soit à l'échappement 45. Le distributeur 43 est piloté, par un circuit électrique 46, par un boîtier électronique 47 de gestion et de commande pilotant également, par un circuit électrique 48, l'appareil de mesure 40. Un circuit électrique 49 relie le boîtier 47 à l'analyseur d'image 35. Les moyens de mémorisation 26a de l'unité de traitement 26 sont reliés par un circuit 51 au moyen de mesure 40.

Après mise en place du brassard 2, et repérage sur l'écran 28 du vaisseau à mesurer, par déplacement de la sonde 21 sur la fenêtre 10, l'opérateur actionne un organe de déclenchement du cycle de mesure, tel qu'un bouton de commande 50 du boîtier 47.

Pour la mesure de la pression sanguine dans le vaisseau dès la reprise de la pulsabilité et avant la reprise du flux sanguin, se succèdent alors les phases suivantes :
- mise en compression du membre par gonflage de la poche gonflable 10 par le circuit 42, alimenté en fluide par le distributeur 43,
- mise en décompression lente par le distributeur 43,
- analyse de l'image de l'écran 27 par l'analyseur 35 jusqu'à perception des pulsations annonciatrices de l'ouverture du vaisseau,
- déclenchement de l'appareil de mesure 40,
- mémorisation dans la mémoire 26a de la valeur mesurée par l'appareil 40.

Avec un échographe évolué, la mesure peut continuer automatiquement, pour mesurer la pression dans d'autres états de référence du vaisseau et, par exemple, jusqu'à la mesure de la pression sanguine au retour du flux sanguin. Dans ce cas, l'analyseur d'image 35 redéclenche, à travers le boîtier 47, l'appareil de mesure de pression 40 dès le retour de la circulation sanguine.

La mémorisation des valeurs mesurées, combinée avec le déplacement du brassard sur la zone de mesure, permet d'obtenir, en fin d'observation, une cartographie du membre indiquant, pour chaque zone de mesure, les valeurs mesurées.

Cette technique de mesure de la pression artérielle et veineuse par méthode indirecte est tout à fait nouvelle, précise et repose sur des bases physiques et facilement reproductibles.

Elle est réalisable sur toute la longueur d'un membre et permet des mesures segmentaires et étagées.

Par l'analyse de la circonférence ou du diamètre du vaisseau, de la différence des pressions à plusieurs niveaux et, dans une forme plus élaborée, de l'analyse de la vitesse des flux, il est possible d'étudier des débits volumiques instantanés, ceci de manière non traumatique et non sanglante.

Les conséquences pour les mesures de pressions artérielles périphériques sont de mieux connaître l'état circulatoire apportant l'oxygène à tous les niveaux explorés et de pouvoir faire une cartographie étagée des pressions et des débits.

Par la mesure des pressions veineuses, cette méthode donne la possibilité de l'appliquer à toutes les veines d'un membre (profondes, superficielles, perforantes, musculaires), à plusieurs niveaux du membre et en différentes positions (couché, assis, debout). Comme pour l'analyse des artères, elle permet de déterminer des débits instantanés et de manière facilement reproductible, selon différentes positions et sans traumatisme.

Ceci est très novateur, car aucune des techniques actuelles ne permet de déterminer des pressions veineuses et des débits instantanés, que ce soit par voie sanglante ou de manière indirecte.

Cette méthode permet de définir une cartographie précise de ces pressions artérielles et veineuses et des débits dans tous les vaisseaux d'un membre, à plusieurs niveaux, selon différentes positions, au repos, au cours d'un effort, pendant la période de récupération après l'effort, etc...

Elle permet de mieux préciser l'état vasculaire et de prendre des décisions plus adaptées pour la prévention et le traitement médical ou chirurgical.

L'échographe peut, sans sortir du cadre de l'invention, être remplacé par tout autre appareil d'observation non invasive du corps humain, par ondes réfléchies ou rayonnements traversants, tel que IRM, Scanner, laser, sous réserve que cet appareil comporte des moyens de traitement des signaux pouvant coopérer avec les moyens complémentaires de l'invention.

## Revendications

1. Procédé de mesure de la pression sanguine d'un vaisseau comprenant une phase de mise en compression du vaisseau par un moyen fluidique associé à un moyen de mesure de la pression du fluide générateur de la compression, une phase de décompression lente et une phase de mesure, le procédé comprenant:
- avant la phase de mise en compression du vaisseau sanguin, une phase de repérage d'une image en au moins deux dimensions du vaisseau, l'image étant générée sur l'écran de lecture (28) d'un appareil d'observation du corps humain par ondes réfléchies ou rayonnements traversants,
- et, pendant l'une des phases, respectivement de compression ou de décompression de ce vaisseau, une phase de repérage à l'écran (28) d'une image en au moins deux dimensions d'un état particulier du vaisseau, cette phase déclenchant, dès que le vaisseau sanguin est dans cet état particulier, la phase de mesure de la pression des moyens fluidiques (5, 6, 7, 43, 44) s'opposant à la pression sanguine instantanée dans le vaisseau.

2. Procédé selon la revendication 1, **caractérisé en ce qu'**il comprend, pendant la phase de compression, une phase de repérage à l'écran de lecture de la fermeture complète du vaisseau, cette dernière phase déclenchant la phase de mesure de la pression des moyens de compression fluidique indiquant la valeur de la pression d'occlusion du vaisseau comprimé.

3. Procédé selon la revendication 1, **caractérisé en ce qu'**il comprend, pendant la phase de décompression lente, une phase de repérage sur l'écran de lecture (28) de la pulsation sanguine précédant l'ouverture du vaisseau et la reprise de l'écoulement sanguin, cette dernière phase déclenchant la phase de mesure de la pression des moyens de compression fluidique, indiquant la valeur de la pression d'occlusion du segment de vaisseau comprimé examiné à la reprise de la pulsabilité.

4. Procédé selon la revendication 1, **caractérisé en ce qu'**il comprend également, pendant la phase de décompression lente, et après la phase de mesure de la pression du vaisseau sanguin comprimé à la reprise de la pulsabilité, une phase de repérage à l'écran de lecture (28) de la reprise de la circulation sanguine, cette phase déclenchant une phase de mesure de la pression de retour du flux sanguin.

5. Procédé selon la revendication 1, **caractérisé en ce qu'**il comprend, après une mesure de la pression d'occlusion du vaisseau sanguin, et à la fin d'une phase de décompression lente, une phase de repérage à l'écran (28) du vaisseau, une phase de compression du vaisseau jusqu'à réduction de son calibre d'une valeur définie, et une phase de mesure de la pression des moyens de compression fluidique, déclenchée quand le vaisseau atteint le calibre attendu.

6. Procédé selon la revendication 5, **caractérisé en ce que** la phase de mesure de la pression sanguine pour un calibre déterminé du vaisseau est complétée par une phase de mesure de la vitesse du flux sanguin dans le vaisseau.

7. Procédé selon l'une quelconque des revendications 1 à 6, **caractérisé en ce qu'**il consiste à mémoriser la valeur de chaque mesure de pression, à recommencer l'opération de mesure en plusieurs zones successives du membre examiné et, en fin de mesure, à appeler à l'écran (28) les différentes valeurs mémorisées pour dresser une cartographie du vaisseau dans le membre examiné, et à mémoriser cette cartographie.

8. Dispositif de mesure de la pression sanguine comprenant un brassard (1) apte à entourer le membre dont le circuit sanguin est soumis à la mesure, ce brassard étant muni, d'une part, d'une poche gonflable (5) raccordée à une source de fluide sous pression (7, 44) par un circuit de fluide (6, 42) équipé d'un moyen de mise à l'échappement (8a, 43) et d'un moyen de mesure de la pression du fluide (9, 40), et d'autre part, d'une fenêtre acoustique (10) qui, réalisée dans un matériau perméable aux ondes ou rayonnements d'un appareil d'observation du corps humain, est apte à être appliquée sur la zone du membre comportant le vaisseau sanguin dont il faut mesurer la pression, ledit appareil étant équipé de moyens de traitement des signaux réfléchis et d'un écran de visualisation (28), et un analyseur d'images (35) apte a) à détecter sur l'écran (28) une image proche d'une image mémorisée correspondant à un état du vaisseau sanguin, tel que début de l'occlusion par compression, compression avec début de pulsabilité du vaisseau, reprise du flux sanguin, mise à un calibre du vaisseau, et b) à commander le fonctionnement d'un appareil de mesure de la pression du fluide dans la poche gonflable, **caractérisé en ce que** les moyens de traitement (26) de l'appareil d'observation comprennent des moyens générant sur l'écran une image en au moins deux dimensions de la zone du vaisseau sanguin soumis aux ondes ou rayonnement de l'appareil.

9. Dispositif selon la revendication 8, **caractérisé en ce qu'**il comprend :
- un boîtier électronique de gestion et de commande (47) réagissant à un organe (50) de déclenchement d'un cycle de mesure, ce cycle comprenant une ou plusieurs mesures successives mais relatives chacune à un état différent du vaisseau,
- un distributeur de fluide (43) piloté par le boîtier de commande (47) et régissant l'alimentation en fluide de la poche gonflable (5) en la mettant en relation soit avec une source de fluide sous pression (44), soit avec un circuit d'échappement (47),
- un analyseur d'écran (35) envoyant au boîtier de commande (47), et quand le vaisseau atteint l'état de référence, un signal déclenchant une phase de la mesure,
- un appareil de mesure (40) de la pression, piloté par le boîtier de commande (47) pour mesurer, dans chaque phase de mesure, la pression dans la poche gonflable (5),
- et des moyens mémorisant la valeur de la pression mesurée.

## Claims

1. Method for measuring the blood pressure of a vessel, comprising a phase of compressing the vessel by a fluidic means associated with a means for measuring the pressure of the fluid generating the compression, a slow decompression phase and a measurement phase, the method comprising:
- before the phase of compressing the blood vessel, a phase of identifying an at least two-dimensional image of the vessel, the image being generated on the readout screen (28) of an apparatus for observing the human body by reflected waves or transmitted radiation,
- and during one of the phases respectively of compressing or decompressing this vessel, a phase of identifying an at least two-dimensional image of a particular state of the vessel on the screen (28), this phase triggering the phase of measuring the pressure of the fluidic means (5, 6, 7, 43, 44) opposing the instantaneous blood pressure in the vessel as soon as the blood vessel is in this particular state.

2. Method according to Claim 1, **characterized in that** during the compression phase it comprises: a phase of identifying complete closure of the vessel on the readout screen, this latter phase triggering the phase of measuring the fluidic compression means pressure indicating the value of the occlusion pressure of the compressed vessel.

3. Method according to Claim 1, **characterized in that** during the slow decompression phase it comprises a phase of identifying the blood pulse preceding the opening of the vessel and the resumption of the blood flow on the readout to screen (28), this latter phase triggering the phase of measuring the fluidic compression means pressure indicating the value of the occlusion pressure of the compressed vessel segment being examined on resumption of the pulsation.

4. Method according to Claim 1, **characterized in that** during the slow decompression phase and after the phase of measuring the pressure of the compressed blood vessel on resumption of the pulsation, it also comprises a phase of identifying the resumption of blood circulation on the readout screen (28), this phase triggering a phase of measuring the return pressure of the blood flow.

5. Method according to Claim 1, **characterized in that** after a phase of measuring the occlusion pressure of the blood vessel and at the end of a slow decompression phase, it comprises a phase of identifying the vessel on screen (28), a phase of compressing the vessel until its calibre is reduced by a defined value and a phase of measuring the pressure of the fluidic compression means, which is triggered when the vessel reaches the expected calibre.

6. Method according to Claim 5, **characterized in that** the phase of measuring the blood pressure for a determined calibre of the vessel is supplemented with a phase of measuring the speed of the blood flow in the vessel.

7. Method according any one of Claims 1 to 6, **characterized in that** it consists in storing the value of each pressure measurement, in recommencing the measurement operation in a plurality of successive zones of the limb being examined and, at the end of measurement, in calling up the various stored values on the screen (28) in order to plot a map of the vessel in the limb being examined and storing this map.

8. Device for measuring blood pressure, comprising a cuff (1) capable enclosing of the limb whose blood circuit is subjected to the measurement, this cuff being provided on the one hand with an inflatable pouch (5) connected to a pressurized fluid source (7, 44) by a fluid circuit (6, 42) equipped with a venting means (8a, 43) and with a means for measuring the pressure of the fluid (9, 40) and on the other hand with an acoustic window (10) which, made of a material permeable to the waves or radiation of an apparatus for observing the human body, is capable of being applied onto the zone of the limb containing the blood vessel whose pressure needs to be measured, the said apparatus being equipped with means for processing the reflected signals and with a display screen (28), and an image analyzer (35) capable of a) detecting an image on the screen (28) similar to a stored image corresponding to a state of the blood vessel such as the start of occlusion by compression, compression with onset of pulsation of the vessel, resumption of the blood flow or the vessel being set to a calibre, and b) controlling the operation of an apparatus for measuring the pressure of the fluid in the inflatable pouch, **characterized in that** the processing means (26) of the observation apparatus comprise means generating an at least two-dimensional image of the zone of the blood vessel subjected to the waves or radiation of the apparatus on the screen.

9. Device according to Claim 8, **characterized in that** it comprises:
- an electronics unit for management and control (47) reacting to an organ (50) for triggering a measurement cycle, this cycle comprising one or more measurements which are successive but each relate to a different state of the vessel,
- a fluid distributor (43) driven by the control unit (47) and governing the fluid supply of the inflatable pouch (5) by setting it in communication either with a pressurized fluid source (44) or with a venting circuit (47),
- a screen analyzer (35) sending a signal triggering a phase of the measurement to the control unit (47) when the vessel reaches the reference state,
- an apparatus (40) for pressure measurement, driven by the control unit (47) in order to measure the pressure in the inflatable pouch (5) during each measurement phase,
- and means storing the value of the measured pressure.

## Patentansprüche

1. Verfahren zur Messung des Blutdrucks eines Gefäßes, umfassend eine Phase der Kompression des Gefäßes durch ein Fluidmittel in Verbindung mit einem Mittel zur Messung des Drucks des Fluids, das den Druck erzeugt, eine Phase der langsamen Dekompression und eine Messphase, wobei das Verfahren Folgendes umfasst:
- vor der Phase der Kompression des Blutgefäßes eine Phase der Lokalisierung eines Bildes des Gefäßes in mindestens zwei Dimensionen, wobei das Bild auf dem Ableseschirm (28) eines Geräts zur Beobachtung des menschlichen Körpers durch reflektierte Wellen oder hindurchgehende Strahlungen erzeugt wird,
- und während einer der Phasen der Kompression bzw. Dekompression dieses Gefäßes eine Phase der Lokalisierung auf dem Schirm (28) eines Bildes in mindestens zwei Dimensionen von einem bestimmten Zustand des Gefäßes, wobei diese Phase von da an, wenn sich das Blutgefäß in diesem bestimmten Zustand befindet, die Phase der Messung des Drucks der Fluidmittel (5, 6, 7, 43, 44), der dem momentanen Blutdruck im Gefäß entgegenwirkt, auslöst.

2. Verfahren nach Anspruch 1, **dadurch gekennzeichnet, dass** es während der Kompressionsphase eine Phase der Lokalisierung des vollständigen Verschlusses des Gefäßes auf dem Ableseschirm umfasst, wobei diese letztere Phase die Phase der Messung des Drucks der Fluidkompressionsmittel auslöst, der den Wert für den Okklusionsdruck des komprimierten Gefäßes angibt.

3. Verfahren nach Anspruch 1, **dadurch gekennzeichnet, dass** es während der langsamen Dekompressionsphase eine Phase der Lokalisierung auf dem Ableseschirm (28) der Blutpulsation, die dem Öffnen des Gefäßes vorausgeht, und der Wiederherstellung des Blutflusses umfasst, wobei diese letztere Phase die Phase der Messung des Drucks der Fluidkompressionsmittel auslöst, der den Wert für den Okklusionsdruck des Segments des komprimierten untersuchten Gefäßes bei Wiederherstellung der Pulsierfähigkeit angibt.

4. Verfahren nach Anspruch 1, **dadurch gekennzeichnet, dass** es ferner während der Phase der langsamen Dekompression und nach der Phase der Messung des Drucks des komprimierten Blutgefäßes bei Wiederherstellung der Pulsierfähigkeit eine Phase der Lokalisierung auf dem Ableseschirm (28) der Wiederherstellung der Blutzirkulation umfasst, wobei diese Phase eine Phase der Messung des Rückkehrdrucks des Blutflusses auslöst.

5. Verfahren nach Anspruch 1, **dadurch gekennzeichnet, dass** es nach einer Messung des Okklusionsdrucks des Blutgefäßes und am Ende einer langsamen Dekompressionsphase eine Phase der Lokalisierung auf dem Schirm (28) des Gefäßes, eine Phase der Kompression des Gefäßes bis zur Verringerung seines Durchmessers um einen definierten Wert und eine Phase der Messung des Drucks der Fluidkompressionsmittel umfasst, die ausgelöst wird, wenn das Gefäß den erwarteten Durchmesser erreicht.

6. Verfahren nach Anspruch 5, **dadurch gekennzeichnet, dass** die Phase der Messung des Blutdrucks bei einem festgelegten Durchmesser des Gefäßes durch eine Phase der Messung der Geschwindigkeit des Blutflusses in dem Gefäß abgeschlossen wird.

7. Verfahren nach einem der Ansprüche 1 bis 6, **dadurch gekennzeichnet, dass** es aus dem Speichern des Wertes jeder Druckmessung, dem erneuten Beginnen der Messung in mehreren aufeinander folgenden Zonen der untersuchten Gliedmaße und, am Ende der Messung, aus dem Aufrufen der verschiedenen gespeicherten Werte auf dem Schirm (28) besteht, um eine Kartografie des Gefäßes in der untersuchten Gliedmaße zu zeichnen und diese Kartografie zu speichern.

8. Vorrichtung zur Messung des Blutdrucks, umfassend eine Armbinde (1), die dazu eingerichtet ist, die Gliedmaße zu umgeben, deren Blutkreislauf der Messung unterzogen wird, wobei diese Armbinde einerseits mit einer aufblasbaren Tasche (5), die mit einer Quelle für unter Druck stehendes Fluid (7, 44) über einen Fluidkreislauf (6, 42) verbunden ist, der mit einem Mittel zum Ablassen (8a, 43) und einem Mittel zur Messung des Fluiddrucks (9, 40) ausgestattet ist, und andererseits mit einem akustischen Fenster (10) ausgestattet ist, das aus einem Material hergestellt ist, das für Wellen oder Strahlungen eines Geräts zur Beobachtung des menschlichen Körpers permeabel ist, und dazu eingerichtet ist, auf die Zone der Gliedmaße, das das Blutgefäß enthält, dessen Druck gemessen werden soll, aufgebracht zu werden, wobei das Gerät mit Mitteln zur Verarbeitung reflektierter Signale und einem Visualisierungsschirm (28) sowie einem Bildanalysator (35) ausgestattet ist, der dazu eingerichtet ist, a) auf dem Schirm (28) ein Bild ähnlich einem gespeicherten Bild zu ermitteln, das einem Zustand des Blutgefäßes entspricht, wie Beginn der Okklusion durch Kompression, Kompression mit Beginn der Pulsierfähigkeit des Gefäßes, Wiederherstellung des Blutflusses, Einstellen eines Durchmessers des Gefäßes, und b) die Funktion eines Gerätes zur Messung des Fluiddrucks in der aufblasbaren Tasche zu steuern, **dadurch gekennzeichnet, dass** die Verarbeitungsmittel (26) des Beobachtungsgerätes Mittel umfassen, die auf dem Schirm ein Bild in mindestens zwei Dimensionen der Zone des Blutgefäßes erzeugen, die den Wellen oder der Strahlung des Gerätes unterworfen wird.

9. Vorrichtung nach Anspruch 8, **dadurch gekennzeichnet, dass** sie Folgendes umfasst:
- ein elektronisches Bedienungs- und Steuerungsgehäuse (47), das auf eine Einrichtung (50) zum Auslösen eines Messzyklus reagiert, wobei dieser Zyklus eine oder mehrere Messungen umfasst, die aufeinander folgen, aber sich jeweils auf einen anderen Zustand des Gefäßes beziehen,
- einen Fluidverteiler (43), der von dem Steuerungsgehäuse (47) geregelt wird und die Versorgung der aufblasbaren Tasche (5) mit Fluid reguliert, indem er sie entweder mit einer Quelle für unter Druck stehendes Fluid (44) oder mit einem Ablasszyklus (47) verbindet,
- einen Bildschirmanalysator (35), der an das Steuerungsgehäuse (47) ein Signal sendet, das eine Messphase auslöst, wenn das Gefäß den Referenzzustand erreicht,
- ein Druckmessgerät (40), das vom Steuerungsgehäuse (47) gesteuert wird, um in jeder Messphase den Druck in der aufblasbaren Tasche (5) zu messen
und Mittel, die den gemessenen Druckwert speichern.
